Europäisches Patentamt

European Patent Office

Office européen des brevets

(11) Publication number: **0 339 905**
**A2**

(12) **EUROPEAN PATENT APPLICATION**

(21) Application number: **89304056.8**

(22) Date of filing: **24.04.89**

(51) Int. Cl.⁴: **A61K 37/02 , A61K 31/07**

(30) Priority: **25.04.88 US 185665**

(43) Date of publication of application:
**02.11.89 Bulletin 89/44**

(84) Designated Contracting States:
**DE FR GB IT**

(71) Applicant: **ETHICON INC.**
**U.S. Route 22**
**Somerville New Jersey 08876(US)**

(72) Inventor: **Sheffield, Warren D.**
**R.D. No. 3 Box 67 Blossom Hill Road**
**Lebanon, NJ 08833(US)**
Inventor: **Mezick, James A.**
**43 Valley Forge Drive**
**East Brunswick, NJ 08816(US)**

(74) Representative: **Fisher, Adrian John et al**
**CARPMAELS & RANSFORD 43 Bloomsbury**
**Square**
**London WC1A 2RA(GB)**

(54) **Wound healing compositions containing growth factors and retinoids.**

(57) Wound healing compositions comprise an effective wound healing amount of at least one polypeptide growth factor having human mitogenic or angiogenic activity and at least one retinoid. Methods of using such compositions for increasing the rate of healing a wound are described.

EP 0 339 905 A2

# WOUND HEALING COMPOSITIONS CONTAINING GROWTH FACTORS AND RETINOIDS

This invention concerns compositions and methods for increasing the rate of healing a wound. The compositions contain at least one growth factor and at least one retinoid.

For many years the medical community has devoted much research time and effort to discovering new and better ways of healing wounds. Some wounds such as burns, ulcers and corneal wounds are difficult to treat and require long periods of time for complete healing. Much research effort has been devoted to discovering new ways of increasing the rate of healing such wounds so as to decrease the time required for healing. At the present time there is still no commercial product available to the public which significantly increases the rate of wound healing. Therefore, there is a need in the medical community for pharmaceutical compositions that can be easily applied to wounds and increase their rate of healing.

Over the past several years, a large amount of attention has been focused on growth factors as agents for use in wound healing. Research in this area has increased at a very fast rate, partially because of the use of recombinant DNA techniques to provide large quantities of the growth factors. Growth factors are natural molecules that encourage the growth of one or more tissues, especially skin, capillary and bone tissue. The growth factors are polypeptides that exert their biological message via a specific plasma membrane receptor in the body.

The human polypeptide growth factors are molecules that regulate the growth of normal human cells. Many human polypeptide growth factors have been identified and their amino acid structures have been determined. See for example, Kris, R.M. et al.,"Growth Factors, Growth Factor Receptors and Oncogenes," BIO/TECHNOLOGY, February 1985, pages 135-140. Some of these growth factors are: epidermal growth factor (EGF), acidic and basic fibroblast growth factor (FGF), platelet-derived growth factor (PDGF), transforming growth factor-$\alpha$ (TGF-$\alpha$), transforming growth factor-$\beta$ (TGF-$\beta$), insulin-like growth factors (IGF-I and IGF-II), and nerve growth factor (NGF). Because of their ability to stimulate cell growth, the human polypeptide growth factors have been described as being useful in stimulating the wound healing process.

One of the better characterized growth factors is epidermal growth factor. Human EGF is a 53 amino acid polypeptide growth factor that has mitogenic activity for a number of kinds of cells, including epithelial and mesenchymal cells. Variants of the naturally occurring EGF molecule have been reported, such as the 52 amino acid gamma-urogastrone. EGF has also been reported to have angiogenic activity. Epidermal growth factor exhibits epidermal growth promoting activity and gastric acid secretion inhibiting activity, and is therefore useful as a medicament. A review of epidermal growth factor can be found in Carpenter, G. et al., "Epidermal Growth Factor, Its Receptor, and Related Proteins," Exp. Cell Res. 164:1-10 (1986).

Besides the growth factors, another class of molecules have been suggested as being effective in promoting wound healing. These are the retinoids. The retinoids have been suggested as being effective in promoting corneal wound healing after surgery in Ubels, J.L. et al., "Healing of Experimental Corneal Wounds Treated With Topically Applied Retinoids," Am.J. Ophthalmol. 95:353-358 (1983). A topical vitamin A ointment has been shown to increase the wound strength in healing corneoscleral incisions in Kastl, P.R. et al, "Topical Vitamin A Ointment Increases Healing of Cataract Incisions," Ann. Ophthal. 19: 175-180 (1987). The retinoids (for example, vitamin A and its derivatives) are substances which are known to have a broad spectrum of biological activity. Retinoids affect the differentiation, maintenance and proliferation of many cell types whether they are of ectodermal, endodermal or mesodermal origin; or whether they are epithelial, fibroblastic or mesenchymal. Past and present uses of retinoids are found in the treatment of severe cystic acne, psoriasis, and other disorders of keratinization. For a review of prior developments in retinoid therapy, see Pawson, B.A. et al., "Retinoids at the Threshold; Their Biological Significance and Therapeutic Potential", J. Med. Chem. 25:1269-1277 (1982). A more recent discussion of retinoids in research and clinical medicine can be found in the publication of a symposium held in Geneva: J.H. Saurat, Editor, "Retinoids, New Trends in Research and Therapy," Karger Publishing Co. (1985).

Several researchers have presented experimental results which suggest that retinoic acid increases the mitogenic activity of epidermal growth factor and its binding to its cell surface receptors in vitro. For example, see Jetten, A.M., "Effects of Retinoic Acid on the Binding and Mitogenic Activity of Epidermal Growth Factor", J. Cell Physiol. 110 (3): 235-40 (1982); Harper, R.A. et al., "Vitamin A Potentiates the Mitogenic Effect of Epidermal Growth Factor in Cultures of Normal Adult Human Skin Fibroblasts", Endocrinology 107 (6): 2113-2114 (1980); and Roberts, A.B. et al., "Antagonistic Actions of Retinoic Acid and Dexamethasone on Anchorage-Independent Growth and Epidermal Growth Factor Binding of Normal Rat Kidney Cells", Cancer Res. 44: 1635-1641 (1984). One investigator has suggested that the effect of retinoids on the binding of EGF to its receptor is that the retinoids increase the number of EGF receptor sites and therefore enables the binding of a greater number of EGF molecules to the receptors. See, Jetten, A.M., "Retinoids Specifically Enhance the Number of Epidermal Growth Factor Receptors", Nature 284: 626-629 (1980); and Jetten, A.M., "Modulation of Cell Growth by Retinoids and Their Possible Mechanisms of Action", Fed. Proc. 43 (1): 134-139 (1984).

Summary of the Invention

The present invention provides wound healing compositions which are superior in promoting the rate of wound healing when compared to the use of a growth factor alone or a retinoid alone. Such wound healing compositions comprise an effective wound healing amount of at least one polypeptide growth factor having human mitogenic or angiogenic activity and at least one retinoid. It is believed that the combination of a growth factor and a retinoid will provide a synergistic response, in that the rate of wound healing after the composition is applied to the wound is greater than the sum of the effect of a growth factor applied to the wound alone and a retinoid applied to the wound alone. Methods for increasing the rate of healing a wound comprise applying the compositions of the present invention to the wound either topically or internally.

Detailed Description of the Invention

It is predicted that the present compositions will have a synergistic effect with respect to growth factors alone and retinoids alone. How this effect is achieved is not known. Without wishing to be bound by theory, one possible explanation is that the retinoids may increase the number of receptors for the growth factor on plasma membranes of the target cells for the growth factor at the wound site, as suggested by prior investigators. If this indeed is the mechanism by which the retinoid acts, then it is likely that any other chemical entity which is capable of increasing the number of receptors for the growth factor will be as effective as a retinoid. Therefore, it is believed that such chemical entities are also within the scope of the present invention.

The polypeptide growth factors that are useful with the present invention are those having human mitogenic or angiogenic activity selected from the group consisting of EGF, acidic FGF, basic FGF, PDGF, TGF-$\alpha$, TGF-$\beta$, angiogenin, NGF, IGF-I and IGF-II. Any combination or mixture of these growth factors may be substituted for an individual growth factor in the compositions. The amino acid structures of these growth factors are well known to those skilled in the art and therefore these molecules are readily identifiable and available. It is contemplated that biologically active fragments or chemically synthesized derivatives of these growth factors may be used instead of the entire naturally occurring molecule. The growth factors may be obtained by isolation from natural sources, chemical synthesis or recombinant DNA techniques. Of course, it is preferred that the growth factor be prepared by recombinant DNA techniques as this provides a more efficient method for providing large amounts of the growth factor. As used herein, the phrase "growth factor" does not include the so called hematopoietic growth factors, such as erythropoietin and the colony stimulating factors.

Retinoids have been defined narrowly as comprising simply vitamin A (retinol) and its derivatives such as vitamin A aldehyde (retinal), vitamin A acid (retinoic acid), comprising the so called natural retinoids. However, subsequent research has resulted in a much larger class of chemical compounds that are termed retinoids due to their biological similarity to vitamin A and its derivatives. Retinoids useful in the present invention include all natural and/or synthetic analogs of vitamin A or retinol-like compounds which possess the biological activity of vitamin A in the skin, such as the control of epithelial cell differentiation of keratinocytes in the epidermis and/or stimulation of fibroplasia or new collagen synthesis in the dermis among other effects. Accordingly, as used herein for the purposes of the present invention, the term "retinoid" will be understood to include any of the foregoing compounds as well as the geometric isomers and stereoisomers of those compounds, such as all-trans retinoic acid and 13-cis-retinoic acid.

The compositions of the present invention may be used in the form of an aqueous medicinal composition such as a buffered solution, gel, cream, suspension, or dispersion. The compositions may also be in the form of an ointment. An effective wound healing amount of the polypeptide growth factor in an aqueous composition is in the range of from about 0.01 to about 1,000 micrograms per milliliter of the aqueous formulation. Preferably, the growth factor concentration is in the range 1-500 micrograms/milliliter and more preferably in the range 1-100 micrograms per milliliter. The concentration of the retinoid in an aqueous formulation is in the range 0.1 to 5,000 micrograms/ml, preferably 1-1,000 micrograms/ml. Expressed on a weight percentage basis (weight of ingredient per total weight of formulation), the retinoid may be present in the formulaion at 0.00001% to 0.5% (w/w), preferably 0.0001% to 0.1%. The exact percentage of the retinoid will depend on the particular retinoid used and its potency. Obviously, the more potent the retinoid the less that will be needed in the formulation. The growth factor may be present at 0.000001% to 0.1% (w/w), preferably 0.0001% to 0.05% and more preferably 0.0001% to 0.01%.

The compositions of the present invention may be formulated in any number of ways depending on whether an aqueous solution, cream or ointment is desired and whether it will be used internally or topically on the surface of the skin or in the eye. A suitable water miscible (substantially oil free and fat free) liquid carrier is a mixture of an alcohol, such as ethyl alcohol or isopropyl alcohol, at 25-75% by weight, and the balance a liquid glycol, such as ethylene glycol or polyethylene glycol.

Compositions formulated as a cream may contain a cream stabilizer, such as xanthan gum; an emulsifier, preferably a non-ionic emulsifier; at least one liquid and one solid hydrophobic material selected from the liquid and solid fatty acids, fatty alcohols, fatty acid esters, pharmaceutical grades of waxes and hydrocarbons, the latter ranging from liquids, through semisolids such as petrolatum, to solids and the like; a preservative; an antioxidant; and water. Suitable non-ionic emulsifiers are the polyoxyalkylene fatty acid esters, in particular the polyoxyalkylene stearates, such as polyoxyethylene 25 oxypropylenestearate, polyoxyl 40 stearate and polyethylene glycol 400 monostearate. The hydrophobic material may be any pharmaceutically acceptable fatty material known and used by those skilled in the art. Such hydrophobic materials include the fatty acids, fatty alcohols and fatty acid esters, wherein the fatty acid moiety has from about 12 to about 20 carbon atoms, such as stearyl alcohol, stearic acid, isopropyl myristate and cetyl alcohol; as well as pharmaceutical grades of beeswax, including white wax, sperm wax, lanolin, mineral oil, etc.

The compositions of the present invention are useful in eye drop formulations, eye gels, eye creams, liposome or micelle formulations, aqueous vehicles for soaking soaked gauze dressings, burn dressings, artificial skins, sutures and staple coatings, salves or creams, gel formulations, foams and the like. Additional material such as buffers, preservatives, tonicity adjusting agents, anti-oxidants, polymers for adjusting viscosity or for use as extenders, and excipients may be used in the compositions. Specific illustrative examples of such other materials include phosphate, citrate, or borate buffers; thimerosal, sorbic acid, methyl or propyl paraben and chlorobutanol preservatives; sodium chloride and/or sugars to adjust the tonicity; and excipients such as mannitol, lactose or sucrose.

Methods for increasing the rate of healing a wound comprise applying or contacting the compositions of the present invention directly to the wound. The composition is permitted to remain in contact with the wound for a period of time sufficient to increase the rate of cell growth at the wound site. Such methods include incorporating any composition of the present invention into a cream formulation or soaking a gauze dressing with an aqueous solution of the composition and then applying the cream or soaked gauze to a wound site such as a burn, donor site wound, ulcer or any type of cutaneous wound. Additionally, sutures or staples may be coated or soaked with the aqueous composition and used to close an open wound.

The types of wounds that may be healed using the compositions of the present invention are those which result from any accidental or medical injury which causes epithelial damage such as ophthalmic wounds, such as those which result from corneal ulcers, radiokeratotomy, corneal transplants, epikeratophakia and other surgically induced wounds in the eye; and cutaneous wounds such as burn wounds, donor site wounds from skin transplants and ulcers (cutaneous, decubitis, venous stasis and diabetic). Additionally, dermatological conditions in which the skin has been damaged, such as psoriasis, sunburn and skin rashes, may be treated with the compositions of the present invention. The compositions may be applied to the wound site either topically or internally, depending on the type of wound.

It is desirable that the compositions of the present invention are formulated into a cream or gel formulation both of which have the advantage of adhering to a wound and conforming to irregular body or wound contours. Gel formulations containing growth factors are described in U.S. Serial No. 98,816, filed September 18, 1987. *

Growth factors have been reported to lose biological activity in the presence of water, and therefore it may be desirable to stabilize the present compositions with a substance that prevents the loss of such biological activity or by lyophilizing the formulations. Stabilized compositions containing growth factors are described in U.S. Patent No. 4,717,717 and U.S. Serial No. 96,455, filed September 18, 1987. * Stable

*See EP-A-0312208

*See EP-A-0267015

**See EP-A-0308238

lyophilized formulations containing growth factors are described in U.S. Serial No. 98,817, filed September 18, 1987. ™

The following examples are presented to illustrate the subject invention. The invention is not to be considered limited by these examples, but only by the appended claims.

## Example 1

A general cream formulation containing epidermal growth factor and all-trans-retinoic acid (tretinoin) is set forth below in Table 1. The EGF is preferred to be human EGF produced by recombinant DNA techniques. Recombinant human EGF may be purchased commercially (for example from Chiron Corporation, Emeryville, CA).

Table 1

| Ingredient | %(w/w) |
|---|---|
| EGF | 0.0001 - 0.05 |
| Tretinoin | 0.0001 - 0.1 |
| Xanthan gum | 0.1 - 1.0 |
| Non-ionic emulsifier | 1.0 - 10.0 |
| Combination of liquid and solid fatty acids, fatty alcohols, fatty acid esters and/or other pharmaceutically acceptable hydrophobic materials | 15.0 - 50.0 |
| Preservative(s) | 0.05 - 1.0 |
| Antioxidant(s) | 0.01 - 1.0 |
| Water | balance to 100.0 |

Optionally, minor amounts of other commonly used cosmetic adjuvants, additives and the like for use as humectants, sequestering agents, dyes, perfume oils and sunscreens may also be included.

## Example 2

A specific cream formulation containing EGF and tretinoin is formulated as set forth in Table 2.

Table 2

| Ingredient | % (w/w) |
|---|---|
| EGF | 0.01 |
| Tretinoin | 0.05 |
| Xanthan gum, food grade | 0.3 |
| Polyoxyl 40 stearate, USP | 5.0 |
| Stearyl alcohol, USP | 3.0 |
| Stearic acid, USP | 19.0 |
| Isopropyl myristate, CTFA | 10.0 |
| Butylated hydroxytoluene FCC | 0.1 |
| Citric acid, USP | 0.05 |
| Sorbic acid, NF | 0.2 |
| Purified water, USP | balance to 100.0 |

Throughout this disclosure, various publications, patents and patent applications are referenced. The disclosures of these publications in their entireties are hereby incorporated by reference into this disclosure in order to more fully describe the state of the art as known to those skilled therein as of the date of the invention described and claimed herein.

The invention has been described herein with reference to certain preferred embodiments and examples. Obvious variations may appear to those skilled in the art. Therefore, the invention is not to be considered limited thereto but only by the claims which follow.

## Claims

1. A composition for increasing the rate of healing a wound, comprising:

a) an effective wound healing amount of at least one polypeptide growth factor having human mitogenic or angiogenic activity; and

b) at least one pharmaceutically acceptable chemical entity which is capable of increasing the number of receptors for the growth factor on the membranes of cells at a wound site.

2. The composition of claim 1 wherein the growth factor is selected from the group consisting of epidermal growth factor, transforming growth factor-alpha, transforming growth factor-beta, basic fibroblast growth factor, acidic fibroblast growth factor, nerve growth factor, insulin-like growth factor, platelet derived growth factor, or any biologically active fragment or derivative thereof.

3. The composition of claim 1, wherein the growth factor is epidermal growth factor.

4. The composition of any of claims 1 to 3, wherein the chemical entity is a retinoid.

5. The composition of claim 4, wherein the retinoid is selected from retinol, retinal, all-trans-retinoic acid and 13-cis-retinoic acid.

6. The use of a polypeptide growth factor having mitogenic or angiogenic activity together with a pharmaceutically acceptable chemical entity which is capable of increasing the number of receptors for the growth factor on the membranes of cells at a wound site, in the manufacture of a medicament for increasing the rate of healing of wounds.